## Europäisches Patentamt

## European Patent Office

## Office européen des brevets

(19)

(11) Veröffentlichungsnummer: **0 015 318**
**B1**

(12) **EUROPÄISCHE PATENTSCHRIFT**

(45) Veröffentlichungstag der Patentschrift:
25.11.81

(51) Int. Cl.³: **C 07 C 33/025**, C 07 C 29/60

(21) Anmeldenummer: 79105031.3

(22) Anmeldetag: 10.12.79

(54) Verfahren zur Herstellung von reinen alpha,omega-C6-bis C20-Alkenolen.

(30) Priorität: 07.02.79 DE 2904518

(43) Veröffentlichungstag der Anmeldung:
17.09.80 Patentblatt 80/19

(45) Bekanntmachung des Hinweises auf die Patenterteilung:
25.11.81 Patentblatt 81/47

(84) Benannte Vertragsstaaten:
BE FR GB IT NL

(56) Entgegenhaltungen:
FR-A-2 262 005
GB-A-1 355 704
GB-A-1 355 705
US-E-19 194
CHEMICAL ABSTRACTS, Band 85,
Nr. 7, 16. August 1976, Seite 471,
Nr. 45 897u
Columbus, Ohio, U.S.A.
L. TOKARZEWSKI et al.: »Studies
on the catalytic dehydration of
hexamethylene glycol«

(73) Patentinhaber: CHEMISCHE WERKE HÜLS AG,
Postfach 1320, D-4370 Marl 1 (DE)

(72) Erfinder: Kaufhold, Manfred, Dr., Bitterfelder Strasse 7a,
D-4370 Marl (DE)
Erfinder: Jacquemin, Werner, Dr., Stockwieser Kamp 12,
D-4358 Haltern (DE)

0 015 318

Verfahren zur Herstellung von reinen $\alpha,\omega$-C$_6$- bis C$_{20}$-Alkenolen

Die Erfindung betrifft ein Verfahren zur Herstellung von reinen $\alpha,\omega$-C$_6$- bis C$_{20}$-Alkenolen durch katalytische Dehydratisierung der entsprechenden $\alpha,\omega$-C$_6$- bis C$_{20}$-Diole.

Die Dehydratisierung von Alkoholen ist eine bekannte, auch technisch angewandte Methode zur Herstellung von Olefinen bzw. allgemein zur Einführung einer Doppelbindung in ein Molekül (Ullmanns Enzyklopädie der technischen Chemie, Band 10 (1958), Seite 41 und Band 8 (1957), Seiten 695 und 696). Es ist weiter bekannt, daß die Dehydratisierung am leichtesten bei tertiären Alkoholen, schwerer bei sekundären und nur unter extremen Bedingungen bei primären Alkoholen durchgeführt werden kann. Bei der Dehydratisierung sowohl in flüssiger als auch in der Gasphase tritt bei Temperaturen über 350° C in Gegenwart von sauren und von festen Katalysatoren teilweise eine Verschiebung der Doppelbindung ein, und zwar mit steigenden Reaktionstemperaturen in größerem Maße.

Aus der US-PS 19 194 war die Abspaltung einer sekundären Hydroxylgruppe im 7,18-Stearyl-Glykol mit Hilfe von starken Säuren wie Chloressigsäure bekannt. Wie bereits ausgeführt, führt die Dehydratisierung mit Hilfe von sauren Katalysatoren zu Isomerisierungen. Die gleichen Nachteile hat die Dehydratisierung von Hexandiol-1,6 über dem sauren Festkontakt Dinatriumhydrogenpyrophosphat (Na$_2$H$_2$P$_2$O$_7$, CA 85 (1976), Seite 471, Nr. 45 897u).

Nach dem Verfahren der US-PS 2 086 713 erhält man durch Dehydratisierung von Diolen, wie z. B. 1,12-Octadekandiol an Hydroxiden des Aluminiums, Zirkoniums, Titans oder Thoriums als Katalysatoren nicht näher definierte ungesättigte Alkohole. Auch bei diesem Verfahren sind Ausbeuten und Selektivität unzureichend.

Nach dem Verfahren der FR-A-2 262 005 sowie der GB-PS 1 355 705 werden Pyrophosphat-Katalysatoren insbesondere zur Dehydratisierung von vicinalen Diolen mit sekundären Hydroxylgruppen eingesetzt. Nach diesen Verfahren erhält man jedoch als Hauptprodukt Diene und ungesättigte Alkohole nur als Nebenprodukte. Die Selektivität bei der Dehydratisierung ist schlecht. Zudem war es aus der DE-AS 2 117 444 bekannt, daß ein zur Dehydratisierung von vicinalen Diolen geeigneter Katalysator nicht bzw. weniger zur Dehydratisierung von nicht vicinalen Diolen geeignet ist.

Sollen einheitliche isomerenfreie Produkte hergestellt werden, ist dieses Auftreten von Isomeren im Reaktionsprodukt ein schwerwiegendes Problem. Es wurden daher verschiedene Methoden zur Verhinderung der Isomerisierung vorgeschlagen. Bei der Interpretation älterer Arbeiten ist aber zu beachten, daß die zu dieser Zeit bekannten analytischen Methoden oft keine genaue Charakterisierung der Reaktionsprodukte zuließen (Houben-Weyl, Methoden der organischen Chemie, Band V/1b, Alkene, Cycloalkene, Arylalkene (1972), Seite 45 ff.).

Das wirkliche Ausmaß der Isomerisierung kann man nur mit exakten analytischen Methoden, z. B. mit Hilfe von gaschromatographischen Analysen, feststellen. Literaturangaben, die nicht auf solchen genauen Analysenwerten fußen, können nichts über die Selektivität der Reaktion aussagen. Da die Isomerisierung durch Säuren bzw. durch saure Zentren auf den Festkontakten katalysiert wird, tritt sie besonders bei der Dehydratisierung von primären Alkoholen auf, die zur Eliminierung des Wassers drastische Bedingungen, d. h. Säuren und hohe Temperaturen benötigen. Alle bisher bekannten Verfahren zur Dehydratisierung von Alkoholen unter Vermeidung der Isomerisierung zielen deshalb darauf ab, bei höheren Reaktionstemperaturen keine starken Säuren einzusetzen und beim Einsatz von Festkontakten die sauren Zentren mit Basen zumindest partiell zu neutralisieren. Geeignete Basen zur Abstumpfung des zu sauren Charakters des Katalysators sind beispielsweise Stickstoffverbindungen wie Ammoniak, tert.-Amine, Pyridine, aber auch Natronlauge und Sodalösung. Diese partielle Neutralisation von Festkontakten hat jedoch auch große Nachteile. Je stärker basisch die zum Abstumpfen verwendete Verbindung ist, um so mehr wird zwar die Isomerisierung unterdrückt, um so größer ist aber auch die Aktivitätsabnahme des Katalysators. Die größere Reinheit des Reaktionsproduktes muß man daher mit langen Reaktionszeiten erkaufen. Ein weiterer Nachteil dieser partiell neutralisierten Katalysatoren ist die kürzere Katalysatorlaufzeit.

Die Dehydratisierung von Amylalkohol ergibt beispielsweise in Gegenwart von Tricalciumphosphat bei 440° C Pentene in einer Ausbeute von 86,9% mit einem Gehalt an Penten-1 von 20,2%. Nach der Behandlung des Katalysators mit Natronlauge steigt der Gehalt an Penten-1 zwar auf 97,2% an, die Ausbeute an Pentenen geht jedoch auf 74,6% zurück. Zudem muß der mit Natronlauge behandelte Katalysator bereits nach 100 Stunden regeneriert werden, da seine Aktivität zu stark abgenommen hat (Chem. Abstr. 58 2353 (1963)). Derartige Verfahren mit Katalysatorlaufzeiten von wenigen Tagen sind unwirtschaftlich und werden deshalb technisch nicht realisiert.

Alle bekannten Verfahren zeigen somit erhebliche Nachteile auf, indem sie entweder mit aktiven Katalysatoren bei hohen Raum-Zeit-Ausbeuten zu uneinheitlichen Produkten führen oder mit partiell neutralisierten Katalysatoren reine Substanzen bei unwirtschaftlicher Arbeitsweise liefern.

Für die Herstellung von $\alpha,\omega$-C$_6$- bis C$_{20}$-Alkenolen durch Dehydratisierung von $\alpha,\omega$-Diolen stellt sich die Aufgabe, ein Verfahren zu finden, das mit minimalem technischen Aufwand und langen Katalysatorlaufzeiten arbeitet und eine so hohe Selektivität besitzt, daß wenig Nebenprodukte und reine, d. h. mindestens 85%ige $\alpha,\omega$-Alkenole gebildet werden. Besonders die Dehydratisierung dieser bifunktionellen Verbindungen stellt hohe Ansprüche an die Selektivität.

Diese Aufgabe wird erfindungsgemäß dadurch gelöst, daß man $\alpha,\omega$-$C_6$- bis $C_{20}$-Diole mit Hilfe von neutralen, einfachen oder Misch-Pyrophosphaten des Lithiums, Natriums, Strontiums oder Bariums bzw. Gemischen dieser Verbindungen als Katalysatoren bei Temperaturen von 300 bis 500°C selektiv und partiell zu reinen $\alpha,\omega$-$C_6$- bis $C_{20}$-Alkenolen mit Reinheitsgraden über 85%, beispielsweise ermittelt durch gaschromatographische Analyse, dehydratisiert und durch Regulierung der Verweilzeit einen Umsatz an Diol von 10 bis 90% einstellt.

Überraschenderweise erhält man bei der Dehydratisierung der $\alpha,\omega$-$C_6$- bis $C_{20}$-Diole, statt der zu erwartenden Diene, in guten Ausbeuten $\alpha,\omega$-$C_6$- bis $C_{20}$-Alkenole mit hohen Reinheitsgraden.

Die Neutralität bezieht sich auf den Katalysator als Ganzes. Denn auch bei den »neutralen« Katalysatoren sind stets noch Zentren mit schwach sauren Eigenschaften vorhanden (Houben-Weyl, Band V/1b, Seiten 47 und 48).

Neutrale, einfache oder Misch-Pyrophosphate des Lithiums, Natriums, Strontiums oder Bariums sind bereits als Dehydratisierungskatalysatoren aus der DE-PS 2 117 444 (=GB-PS 1 355 704 und 1 355 705) bekannt, in der hervorgehoben wird, daß diese Katalysatoren zur Dehydratisierung von vizinalen Diolen mit sekundären Hydroxylgruppen geeignet sind, während zur Dehydratisierung anderer Diole mit primären Hydroxylgruppen saure Katalysatoren eingesetzt werden. Überraschenderweise wurde jedoch gefunden, daß neutrale, einfache oder Misch-Pyrophosphate des Lithiums, Natriums, Strontiums oder Bariums oder Gemische dieser Verbindungen geeignet sind zur selektiven und partiellen Dehydratisierung von $\alpha,\omega$-$C_6$- bis $C_{20}$-Diolen zu $\alpha,\omega$-$C_6$- bis $C_{20}$-Alkenolen. Bei Temperaturen von 300 bis 500°C, vorzugsweise 380 bis 450°C, erhält man $\alpha,\omega$-$C_6$- bis $C_{20}$-Alkenole mit einer beispielsweise gaschromatographisch ermittelten Reinheit von über 85% und nur 2 bis 10% Isomerisierungsprodukte. Besonders geeignet ist Barium-Pyrophosphat, mit dem man die $\alpha,\omega$-$C_6$- bis $C_{20}$-Alkenole in einer Reinheit von über 92% erhält.

Aus den Reaktionsprodukten läßt sich leicht erkennen, ob ein insgesamt neutraler Katalysator saure Zentren besitzt oder nicht, da das Ausmaß an Isomerisierung um so größer ist, je saurer der Katalysator ist. Am Beispiel II der DE-PS 2 117 444 (=GB-PS 1 355 704, Example 2), bei dem der Gehalt an Alkohol im Reaktionsaustrag größer ist als bei den anderen Beispielen, wird deutlich, daß in hohem Maß Isomerisierung stattgefunden hat, denn 76% 2-Methyl-1-butenol-(3) stehen insgesamt 14,4% Isomerisierungsprodukte gegenüber, beispielsweise das durch Allylumlagerung entstandene 3-Methyl-2-butenol-(1). Das Beispiel V dieser Patentschrift zeigt, daß die Isomerisierung bei Temperaturerhöhung von 400 auf 500°C stark zunimmt. Aufgrund dieser Angaben war anzunehmen, daß $\alpha,\omega$-$C_6$- bis $C_{20}$-Diole mindestens ebensoviele Isomerisierungsprodukte liefern würden wie die vizinalen Diole.

Da primäre Hydroxylgruppen nicht so leicht eliminierbar sind wie sekundäre, sind für den Einsatz der neutralen, einfachen oder Misch-Pyrophosphate hohe Temperaturen erforderlich. Diese sollten eine selektive Reaktionsführung erschweren. Überraschenderweise wurde jedoch gefunden, daß trotz der hohen Temperaturen die Reaktion viel selektiver verläuft als die in den Beispielen der DE-PS 2 117 444 (=GB-PS 1 355 704) beschriebenen Dehydratisierungen von sekundären Alkoholen.

Die als Katalysatoren geeigneten neutralen, einfachen oder Misch-Pyrophosphate weisen hohe Aktivitäten auf, die sie noch bei Laufzeiten von über einem Monat behalten. Dies sind Standzeiten, die mit partiell neutralisierten Katalysatoren nicht erreichbar sind.

Im Gegensatz zum Verfahren der DE-PS 2 117 444 (=GB-PS 1 355 704) arbeitet man beim erfindungsgemäßen Verfahren mit Umsätzen unter 100%, von 10 bis 90%, vorzugsweise von 40 bis 80%. In diesem Bereich werden überraschend gute Ausbeuten an $\alpha,\omega$-$C_6$- bis $C_{20}$-Alkenolen von 50 bis 90% erzielt, bei einem sehr niedrigen Anfall an unerwünschtem Dien mit Konzentrationen von 0,5 bis 2% im Reaktionsprodukt. Der Umsatz an Diol von 40 bis 80% ist bevorzugt, da die Wirtschaftlichkeit des Verfahrens in diesem Bereich wegen hoher Raum-Zeit-Ausbeute hoch ist. Bei Umsätzen über 80% nimmt die Dien-Bildung langsam, bei Umsätzen über 90% stark zu. Man ermittelt, gegebenenfalls in einem Vorversuch, gaschromatographisch den Umsatz und stellt nach den ermittelten Umsätzen Verweilzeit und Temperatur ein.

Die hohen Ausbeuten an $\alpha,\omega$-$C_6$- bis $C_{20}$-Alkenolen bei der Dehydratisierung der $\alpha,\omega$-$C_6$- bis $C_{20}$-Diole überraschen insbesondere, da $\alpha,\omega$-Diole zahlreiche Nebenreaktionen eingehen können. So bilden sie leicht cyclische Ether und hochmolekulare Polyether. Aus Butandiol-1,4 entsteht beispielsweise mit Hilfe dieser Dehydratisierungskatalysatoren fast nur Tetrahydrofuran (s. Vergleichsbeispiel 1), aus Pentandiol-1,5 in hohem Anteil Tetrahydropyran (s. Vergleichsbeispiel 2).

Die nach dem erfindungsgemäßen Verfahren erhaltenen $\alpha,\omega$-Alkenole mit 6 bis 20 C-Atomen sind wertvolle Zwischenprodukte für zahlreiche weitere technische Synthesen.


Beispiel 1 (Vergleichsbeispiel)


Ein Quarzrohr, $30 \times 500$ mm, mit elektrischer Mantelheizung füllt man mit 250 ml Bariumpyrophosphat, hergestellt nach den Angaben der DE-PS 2 177 444, Beispiel 2 (=GB-PS 1 355 704, Example 2). Bei einer Kontakttemperatur von 380°C tropft man aus einem Tropftrichter 87 g/h Butandiol-1,4 auf den Kontakt. Gleichzeitig gibt man 23 Nl/h Stickstoff auf den Kopf des Kontaktrohres. Der Umsatz an Butandiol-1,4 beträgt 74%, die Ausbeute an Tetrahydrofuran 72% d. Th. und an Buten-1-ol-4 3% d. Th. Den Ofenaustrag von 237 g destilliert man an einer 25 × 200-mm-Multifilkolonne mit Wasserauskreiser und erhält folgende Fraktionen:

| Fraktion Nr. | Kp °C | Druck mbar | Menge g |
|---|---|---|---|
| Kühlfalle | | | 31 |
| 1 | 63 bis 70 | 1010 | 111 Tetrahydrofuran |
| 2 | 70 bis 130 | 1010 | 45 40 g H$_2$O, 5 g Butenole |
| 3 | 132 bis 133 | 24 | 67 Rück-Butandiol |
| Rückstand | | | 14 |

### Beispiel 2 (Vergleichsbeispiel)

Man setzt das in Beispiel 1 beschriebene Quarzrohr mit der gleichen Kontaktfüllung ein. Bei einer Kontakttemperatur von 430°C tropft man aus einem Tropftrichter 173 g/h Pentandiol-1,5 auf den Kontakt. Gleichzeitig gibt man 45 Nl/h Stickstoff auf den Kopf des Kontaktrohres. Den einphasigen Ofenaustrag von 415 g destilliert man an einer 25 × 500-mm-Multifilkolonne mit Wasserauskreiser. Man erhält folgende Fraktionen:

| Fraktion Nr. | Kp °C | Druck mbar | Menge g |
|---|---|---|---|
| 1 | 75 | 1010 | 70 H$_2$O/Öl Azeotrop |
| 2 | 80 bis 100 | 1010 | 96 Tetrahydropyran |
| 3 | 50 bis 70 | 26 | 107 Pentenole |
| 4 | 70 bis 145 | 26 | 109 Rück-Pentandiol |
| Rückstand | | | 27 |

Die Fraktion 3 besteht nach GC-Analyse zu 92,1% aus Penten-1-ol-5. Das Destillationsergebnis zeigt, daß neben der Dehydratisierung zu Pentenolen fast in gleichem Ausmaß eine cyclisierende Dehydratisierung zu Tetrahydropyran erfolgt.

Der Umsatz an Pentandiol-1,5 beträgt 79%, die Ausbeute an Pentenol 32% d. Th. und an Tetrahydropyran 28% d. Th.

### Beispiel 3

In der in Beispiel 1 beschriebenen Apparatur mit der gleichen Kontaktfüllung tropft man bei einer Kontakttemperatur von 410°C aus einem Tropftrichter 87 g/h Hexandiol-1,6 auf den Kontakt. Gleichzeitig gibt man 23 Nl/h Stickstoff auf den Kopf des Kontaktrohres. Der gaschromatographisch ermittelte Umsatz beträgt 65%.

Den Ofenaustrag von 245 g destilliert man an einer 25 × 500-mm-Multifilkolonne mit Wasserauskreiser und erhält folgende Fraktionen:

# 0 015 318

| Fraktion Nr. | Kp °C | Druck mbar | Menge g |
|---|---|---|---|
| Kühlfalle | | 13 | 20 $H_2O$ |
| Kühlfalle | | 13 | 25 Hexadien |
| 1 | 64 bis 65 | 13 | 98 Hexenole |
| 2 | 135 bis 142 | 13 | 91 Rück-Hexandiol |
| Rückstand | | | 3 |

Die Fraktion 1 wurde gaschromatographisch analysiert.

| Vorlauf | 0,9 Gewichtsprozent |
|---|---|
| Hexanol | 1,8 Gewichtsprozent |
| Hexen-1-ol-6 | 92,5 Gewichtsprozent |
| trans-Hexen-2-ol-6 | 1,7 Gewichtsprozent |
| cis-Hexen-2-ol-6 | 0,8 Gewichtsprozent |
| Rest | 2,3 Gewichtsprozent |

Der Hexandiol-1,6-Umsatz beträgt 65%, die Ausbeute an Hexen-1-ol-6 (ger. 100%) 62,2% d. Th.


## Beispiel 4

Einen 650-ml-V2A-Ofen mit elektrischer Mantelheizung füllt man mit 600 ml Bariumpyrophosphat. Bei einer Kontakttemperatur von 430°C tropft man 180 g/h Decandiol-1,10 auf den Kopf des Ofens zu. Gleichzeitig führt man 60 Nl/h Stickstoff durch den Ofen. Der gaschromatographisch ermittelte Umsatz beträgt 68%.

Den Ofenaustrag destilliert man anschließend und erhält folgende Fraktionen:

| Wasser | 7,2 Gewichtsprozent |
|---|---|
| Decadien | 6,0 Gewichtsprozent |
| Decenole | 47,6 Gewichtsprozent |
| Rück-Decandiol | 32,6 Gewichtsprozent |
| Rückstand | 6,5 Gewichtsprozent |

Die gaschromatographische Analyse der Decenol-Fraktion ergibt:

| Vorläufe | 0,25 Gewichtsprozent |
|---|---|
| Decanol | 3,0 Gewichtsprozent |
| Decen-1-ol-10 | 95,1 Gewichtsprozent |
| trans-Decen-2-ol-10 | 0,7 Gewichtsprozent |
| cis-Decen-2-ol-10 | 0,2 Gewichtsprozent |
| Rest | 0,75 Gewichtsprozent |

Der Decandiol-Umsatz beträgt 68%, die Ausbeute an Decen-1-ol-10 (ger. 100%) 69,4% d. Th.
Nach einer Laufzeit von 30 Tagen hat der Katalysator noch die gleiche Aktivität und Selektivität.


## Beispiel 5

Nach den Angaben der Beispiele 1 bis 3 werden bei einer Kontakttemperatur von 425°C 85 g/h Dodecandiol-1,12 und 23 Nl/h Stickstoff durch den Ofen gefahren. Der gaschromatographisch ermittelte Umsatz beträgt 67%. Die Fraktionierung des Ofenaustrages ergibt:

| Dodecadiene | 13,9% |
|---|---|
| Dodecenole | |
| (Kp/24 mbar 145 bis 149°C) | 49,1% |
| Rück-Dodecandiol | 22,4% |
| Rückstand | 4,6% |

5

Die gaschromatographische Analyse der Dodecenol-Fraktion ergibt:

| | |
|---|---|
| Vorlauf | 0,5 Gewichtsprozent |
| Dodecanol | 4,2 Gewichtsprozent |
| Dodecen-1-ol-12 | 92,2 Gewichtsprozent |
| trans-Dodecen-2-ol-12 | 1,8 Gewichtsprozent |
| cis-Dodecen-1-ol-12 | 0,5 Gewichtsprozent |
| Rest | 0,6 Gewichtsprozent |

Der Umsatz an Dodecandiol-1,12 beträgt 73%, die Ausbeute an Dodecen-1-ol-12 (ger. 100%) 50% d. Th.

## Beispiel 6

### Dehydratisierung von Tetradecandiol-1,14

| | |
|---|---|
| Apparatur | wie Beispiel 1 |
| Kontaktfüllung | wie Beispiel 1 |

Zulauf aus beheiztem Tropftrichter auf den Kopf des Kontaktrohres.

Durch den Ofen werden bei 410°C Kontakttemperatur 120 g/h Tetradecandiol-1,14 und 40 Nl/h Stickstoff gefahren.

Der Ofenaustrag wird an einer 25 × 200-mm-Multifilkolonne destilliert. Einsatz zur Destillation: 420 g. Die Fraktionierung des Ofenaustrags ergibt:

| | |
|---|---|
| Tetradecadiene | 14,2% |
| Tetradecenol | 46,7% |
| Rück-Tetradecandiol | 29,8% |
| Rückstand | 9,3% |

GC-Analyse der Tetradecenol-Fraktion:

| | |
|---|---|
| Vorlauf | 0,4 Gewichtsprozent |
| Tetradecanol | 3,9 Gewichtsprozent |
| Tetradecen-1-ol-14 | 92,4 Gewichtsprozent |
| trans-Tetradecen-2-ol-14 | 1,9 Gewichtsprozent |
| cis-Tetradecen-2-ol-14 | 0,8 Gewichtsprozent |
| Rest | 0,6 Gewichtsprozent |

Der Tetradecandiol-Umsatz beträgt 70%, die Ausbeute an Tetradecen-1-ol-14 (ger. 100%) 66% d. Th.

## Beispiel 7

### Dehydratsierung von Eicosandiol-1,20

| | |
|---|---|
| Apparatur | wie Beispiel 6 |
| Kontakt | wie Beispiel 6 |

Durch den Ofen werden bei 430°C Kontakttemperatur 95 g/h Eicosandiol-1,20 und 35 Nl/h Stickstoff gefahren.

Die Fraktionierung des Ofenaustrags ergibt:

| | |
|---|---|
| Eicosadiene | 12,3% |
| Eicosenol | 48,7% |
| Rück-Eicosandiol | 24,9% |
| Rückstand | 14,1% |

GC-Analyse der Eicosenol-Fraktion:

| Vorlauf | 0,5% |
|---|---|
| Eicosanol | 3,9% |
| Eicosen-1-ol-20 | 93,1% |
| trans-Eicosen-2-ol-20 | 1,4% |
| cis-Eisosen-2-ol-20 | 0,7% |
| Rest | 0,4% |

Der Eicosandiol-Umsatz beträgt 75%, die Ausbeute an Eicosen-1-ol-20 (ger. 100%) 64% d. Th.

## Beispiel 8

Man setzt das in Beispiel 1 beschriebene Quarzrohr ein und füllt es mit 250 ml Lithiumpyrophosphat, hergestellt nach den Angaben der DE-PS 2 117 444, Beispiel 3 ( = GB-PS 1 355 704, Example 3). Bei einer Kontakttemperatur von 450°C tropft man aus einem Tropftrichter 87 g/h Decandiol-1,10 auf den Kontakt. Gleichzeitig gibt man 23 Nl/h Stickstoff auf den Kopf des Kontaktrohres. Der Umsatz an Decandiol-1,10 beträgt 59%, die Ausbeute an Decenolen beträgt 71% d. Th.

Nach der gaschromatographischen Analyse hat das destillierte Decenol folgende Zusammensetzung:

| Decanol-1 | 2,2% |
|---|---|
| Decen-1-ol-10 | 86,9% |
| trans-Decen-2-ol-10 | 4,8% |
| cis-Decen-2-ol-10 | 3,4% |
| Hochsieder | 1,1% |
| Restliche Verbindungen | 1,6% |

## Patentansprüche:

1. Verfahren zur Herstellung von reinen $\alpha,\omega$-C$_6$- bis C$_{20}$-Alkenolen mit Reinheitsgraden über 85% durch katalytische Dehydratisierung der entsprechenden $\alpha,\omega$-C$_6$- bis C$_{20}$-Diole, dadurch gekennzeichnet, daß man $\alpha,\omega$-C$_6$- bis C$_{20}$-Diole mit Hilfe von neutralen, einfachen oder Misch-Pyrophosphaten des Lithiums, Natriums, Strontiums oder Bariums bzw. Gemischen dieser Verbindungen als Katalysatoren bei Temperaturen von 300 bis 500°C selektiv und partiell zu reinen $\alpha,\omega$-C$_6$- bis C$_{20}$-Alkenolen dehydratisiert und durch Regulierung der Verweilzeit einen Umsatz an Diol von 10 bis 90% einstellt.

2. Verfahren nach Anspruch 1, dadurch gekennzeichnet, daß man als Katalysator Barium-Pyrophosphat einsetzt.

## Claims

1. A process for preparing pure $\alpha,\omega$-C$_6$- to C$_{20}$-alkenols of a purity of greater than 85% by catalytically dehydrating the corresponding $\alpha,\omega$-C$_6$- to C$_{20}$-diols, characterized in that $\alpha,\omega$-C$_6$- to C$_{20}$-diols are dehydrated selectively and partially to pure $\alpha,\omega$-C$_6$- to C$_{20}$-alkenols by using a catalyst of a neutral, simple or complex pyrophosphate of lithium, sodium, strontium or barium or a mixture thereof at a temperature of 300—500°C wherein the conversion is controlled within a range of 10—90% by regulating the residence time.

2. The process of Claim 1, wherein barium pyrophosphate is the catalyst.

## Revendications

1. Procédé pour la fabrication d' $\alpha,\omega$-C$_6$- à C$_{20}$-alkénols purs ayant des degrés de pureté dépassant 85% par déshydratation catalytique des $\alpha,\omega$-C$_6$- à C$_{20}$-diols correspondants; caractérisé par le fait que, lors de la déshydratation, des $\alpha,\omega$-C$_6$- à C$_{20}$-diols sont transformés sélectivement et partiellement en $\alpha,\omega$-C$_6$- à C$_{20}$-alkénols purs à l'aide de pyrophosphates neutres, simples ou mixtes, du lithium, du sodium, du strontium ou du baryum ou de mélanges de ces composés en tant que catalyseurs à des températures de 300 à 500°C et qu'un taux de transformation de diol allant de 10 à 90% est ajusté par régulation de la durée du temps de séjour.

2. Procédé conforme à la revendication 1, caractérisé par le fait que l'on utilise comme catalyseur le pyrophosphate de baryum.